**Europäisches Patentamt**

**European Patent Office**

**Office européen des brevets**

(19)

(11) Veröffentlichungsnummer : **0 061 167**
**B1**

(12) # EUROPÄISCHE PATENTSCHRIFT

(45) Veröffentlichungstag der Patentschrift :
17.07.85

(21) Anmeldenummer : 82102257.1

(22) Anmeldetag : 19.03.82

(51) Int. Cl.⁴ : **G 01 N 33/545, A 61 K 41/00**

(54) **Mittel zur immunologischen Diagnose sowie Verfahren zu seiner Herstellung.**

(30) Priorität : 24.03.81 DE 3111474

(43) Veröffentlichungstag der Anmeldung :
29.09.82 Patentblatt 82/39

(45) Bekanntmachung des Hinweises auf die Patenterteilung : 17.07.85 Patentblatt 85/29

(84) Benannte Vertragsstaaten :
AT BE CH DE FR GB IT LI LU NL SE

(56) Entgegenhaltungen :
GB-A- 1 238 280

(73) Patentinhaber : BEHRINGWERKE AKTIENGESELLS-
CHAFT
Postfach 1140
D-3550 Marburg/Lahn (DE)

(72) Erfinder : Ziegelmaier, Robert, Dr.
Renthof 10
D-3550 Marburg/Lahn (DE)

(74) Vertreter : Meyer-Dulheuer, Karl-Hermann, Dr. et al
HOECHST Aktiengesellschaft Zentrale Patentabteilung Postfach 80 03 20
D-6230 Frankfurt/Main 80 (DE)

## Beschreibung

Die Erfindung betrifft ein Mittel zum Nachweis und zur Bestimmung eines in einer Flüssigkeit befindlichen Partners einer immunologischen Reaktion, worin sich der zugehörige Partner auf der Oberfläche eines Trägers befindet, dadurch gekennzeichnet, daß der Träger bestrahlt ist.

Es ist bekannt, einen Partner einer immunologischen Reaktion adsorptiv oder kovalent an einen Träger zu binden, um den entsprechenden Partner aus einer Lösung zu isolieren und diesen dann nachzuweisen oder seine Menge zu bestimmen.

Es ist jedoch auch bekannt, daß auf diesem Prinzip beruhende Testverfahren Mängel aufweisen können.

So beschreiben Chessum und Denmark in The Lancet (1978), Seite 161, daß die in einem allgemein als ELISA (Enzyme Linked Immuno Sorbent Assay) bezeichneten Verfahren zur Bestimmung von Antigenen oder Antikörpern als Träger verwendeten bekannten Mikrotiterplatten aus Polystyrol zu untragbar großen Schwankungen des Testergebnisses führen können. Ebenso berichten Bidwell et al. in J. Infect. Disease 136, Supplement, Seite 274 (1977), über Schwankungen der Ergebnisse eines ELISA-Tests, die auf Unterschiede in den als Träger eingesetzten Mikrotiterplatten zurückgeführt werden konnten. Diese großen, durch Unterschiede in den Oberflächeneigenschaften der Mikrotiterplatten hervorgerufenen Schwankungen des Testergebnisses sollten nicht auf die Verwendung dieser Platten beschränkt sein, sondern auch bei anders geformten, aus denselben Materialien hergestellen Trägern auftreten.

Es bestand demnach die Aufgabe, Trägermaterialien mit gleichmäßigeren Oberflächeneigenschaften zu finden, bei deren Verwendung die Ergebnisse immunologischer Tests weniger variieren.

Überraschend wurde nun gefunden, daß diese Aufgabe dadurch gelöst werden kann, daß das Trägermaterial, vorzugsweise hieraus hergestellte Mikrotiterplatten, mit, vorzugsweise 0,1 bis 100, besonders 0,5 bis 10 Megarad ($0,5 \cdot 10^4$ J/kg-$10^5$ J/kg) Gammastrahlen bestrahlt wird, vorzugsweise mit Cobalt-Strahlen.

Gegenstand der Erfindung ist also ein Verfahren zum Nachweis und zur Bestimmung eines Partners einer immunologischen Reaktion, worin sich der entsprechende Partner auf der Oberfläche eines Trägers befindet, dadurch gekennzeichnet, daß der Träger mit Gammastrahlen bestrahlt wird, bevor oder nachdem seine Oberfläche mit diesem Partner beschichtet wurde.

Es ist nicht erforderlich, den Träger unverzüglich nach dem Bestrahlen zu beschichten. Die vorteilhafte Wirkung einer Bestrahlung wird auch beobachtet, wenn zwischen Bestrahlung und Beschichtung ein Zeitraum von Tagen oder Monaten verstreicht.

Trägermaterialien, die in diesem Verfahren verwendet werden können, sind im Prinzip alle Kunststoffe, insbesondere aber Polystyrol und Polyvinylchlorid.

Bestrahlte Träger können vorteilhaft in an sich bekannten, beispielsweise auf einer Antigen-Antikörper-Reaktion beruhenden Immuntestsystemen eingesetzt werden, beispielsweise ELISA-Systemen (Enzyme Linked Sorbent Assay-Systemen), wobei ein Bindungspartner an den bestrahlten Träger gebunden ist.

Solche Bindungspartner können beispielsweise Proteine wie Immunglobuline, antimikrobielle Antikörper, Plasmaproteine, beispielsweise C1q oder antigen wirksame Materialien, beispielsweise Allergene oder mikrobielle Antigene, sein.

Das folgende Beispiel erläutert die Erfindung :

## Beispiel

Herstellung eines Testbestecks zur Bestimmung von Antikörpern der IgG- und IgM-Klasse gegen Mumpsvirus in Humanserum.

1. Binden der Virusantigens an den Träger (Polystyrol-Mikrotiterplatte)
   a. bestrahlt (etwa 2 Mrad = $2 \cdot 10^4$ J/kg)
   b. unbestrahlt
2. Waschen
3. Auftragen und Inkubieren von Serumverdünnungen
4. Waschen
5. Auftragen und Inkubieren von Anti-Human IgG- oder IgM-alkalische Phosphatase-Konjugat
6. Waschen
7. Auftragen der Substratlösung
8. Abstoppen der enzymatischen Spaltung durch Zugabe von 2N NaOH
9. Photometrieren bei 405 nm

(Siehe Tabelle Seite 3 f.)

# 0 061 167

Tabelle

Vergleich der Empfindlichkeit eines Tests mit bestrahltem mit der eines unbestrahlten Trägers :

| | Nachweisgrenze (Titer) | | | |
|---|---|---|---|---|
| | bestrahlter Träger | | unbestrahlter Träger | |
| Serum | IgG | IgM | IgG | IgM |
| A | 20480 | 20480 | 1280 | 2560 |
| B | 1280 | 160 | 640 | 20 |

Aus den Zahlen ergibt sich der Vorteil einer Verwendung bestrahlter Träger. Die Nachweisfähigkeit (Sensitivität) ist deutlich erhöht.

**Patentansprüche** (für die Vertragsstaaten : BE, CH, DE, FR, GB, IT, LI, LU, NL, SE)

1. Mittel zum Nachweis und zur Bestimmung eines in einer Flüssigkeit befindlichen Partners einer immunologischen Reaktion, wobei sich der zugehörige Partner auf der Oberfläche eines Trägers befindet, dadurch gekennzeichnet, daß der Träger mit Gammastrahlen bestrahlt ist.

2. Verfahren zur Herstellung eines Mittels nach Anspruch 1, in welchem ein Partner eine immunologischen Reaktion auf einen Träger aufgebracht wird, dadurch gekennzeichnet, daß zuvor oder danach mit Gammastrahlen bestrahlt wird.

**Patentanspruch** (für den Vertragsstaat AT)

Verfahren zur Herstellung eines Mittels zum Nachweis und zur Bestimmung eines in einer Flüssigkeit befindlichen Partners einer immunologischen Reaktion, in welchem ein Partner einer immunologischen Reaktion auf einen Träger aufgebracht wird, dadurch gekennzeichnet, daß zuvor oder danach mit Gammastrahlen bestrahlt wird.

**Claims** (for the Contracting States : BE, CH, DE, FR, GB, IT, LI, LU, NL, SE)

1. An agent for the detection and determination of an immunological reaction partner present in a liquid formed by the surface of a carrier with the associated partner being present thereon, wherein the carrier is irradiated with gamma-rays.

2. A process for the preparation of an agent as claimed in claim 1, in which a partner of an immunological reaction is applied to said carrier, wherein the carrier is irradiated beforehand or afterwards with gamma-rays.

**Claim** (for the Contracting State AT)

A process for the preparation of an agent for the detection and determination of an immunological reaction partner present in a liquid, in which a partner of an immunological reaction is applied to a carrier, wherein the carrier is irradiated beforehand or afterwards with gamma-rays.

**Revendications** (pour les Etats contractants : BE, CH, DE, FR, GB, IT, LI, LU, NL, SE)

1. Moyen de détection et de détermination d'un partenaire d'une réaction immunologique, présent dans un liquide, le partenaire correspondant se trouvant sur la surface d'un support, caractérisé en ce que le support est irradié avec des rayons gamma.

2. Procédé pour la préparation d'un moyen selon la revendication 1, dans lequel un partenaire d'une réaction immunologique est appliqué sur un support, caractérisé en ce qu'on irradie avant ou après avec des rayons gamma.

**Revendication** (pour l'Etat contractant AT)

Procédé pour la préparation d'un moyen pour la détection et la détermination d'un partenaire d'une réaction immunologique, présent dans un liquide, dans lequel un partenaire d'une réaction immunologique est appliqué sur un support, caractérisé en ce qu'on irradie avant ou après avec des rayons gamma.

3